# EUROPEAN PATENT SPECIFICATION

(11) **EP 0 727 240 B1**
(45) Date of publication and mention of the grant of the patent: **02.05.2003**
(21) Application number: 96850032.2
(22) Date of filing: 13.02.1996
(51) Int. Cl.: A61N 1/05

(54) **Method for producing an electrode contact device for pacemaker electrode cable application**
Verfahren zur Herstellung einer Elektrodenanordnung für die Verwendung an einem Elektrodenkabel eines Herzschrittmachers
Procédé de production d'un terminal de contact d'une électrode destiné à un câble d'électrodes pour stimulateur cardiaque

(30) Priority: 20.02.1995 SE 9500618
(43) Date of publication of application: 21.08.1996
(73) Proprietor: St. Jude Medical AB, 175 84 Järfälla (SE)
(72) Inventor: Jarl, Per-Erik, 175 63 Järfälla (SE)

(56) References cited:
- WO-A-93/20750
- SE-B- 401 320
- US-A- 4 387 727
- US-A- 4 760 852
- US-A- 4 848 352
- US-A- 4 892 102
- US-A- 5 190 052

## Description

A contact device of the type in question is intended to be mounted at one end of an insulated electrical conductor or cable, and it has the purpose of transmitting electrical signals/electrical current to and from the conductor and the electrical unit or component attached at the end of the conductor.

The contact device to be produced by the method according to the invention may be either an electrode contact head at the distal end of an electrode cable leading to a cardiac pacemaker, or an attachment device through which the other end of the electrode cable, the proximal end, is coupled to the electrode attachment part of a cardiac pacemaker or pacesetter.

A cardiac pacemaker is a battery-operated, electronic impulse generator which can be surgically implanted in a patient whose heart presents dysrhythmia (heart beat irregular or too slow). The impulse generator has the object of stimulating the heart muscle to contract when the heart's own rate of contraction is inadequate. The electrical impulses from such a cardiac pacemaker are transmitted to the heart via thin electrode cables (thin, insulated metal leads) whose distal ends anchored inside the heart, in the cardiac musculature, are designed as electrode contact heads with noninsulated contact portions or zones which are in direct electrical contact with the cardiac musculature. When the pacemaker is, for example, a so-called dual-chamber pacemaker, the latter is in communication with the musculature of the heart via two electrode cables, one of which has its electrode contact head arranged in the right atrium, while the other one has its electrode contact head arranged in the right ventricle. The electrode cables extend into the heart via the subclavian vein. The actual pacemaker is usually surgically implanted under the skin,

The present invention relates quite generally to a method for producing an electrode contact device for medical application as a contact device at one end of an electrically insulated conductor or electrode cable for a cardiac pacemaker.

Such contact device comprises a main body made of ceramic material. On the exterior of the contact device there are at least two separate contact portions each consisting of electrically conductive material and being adapted to be electrically connected with a respective conductor connectable to the contact device.

### Prior art

In DE-A-2319054 there is described a similar type of contact device. Another corresponding type of contact device is described in DE-A-4112936. over the left pectoral muscle.

One object of the invention is to provide a method for producing an electrode contact device with a main body which is made of ceramic material and which can constitute a support for the required number of contact portions (in the form of surface areas or zones) made of electrically conductive material, which contact portions can be produced simply, both with the desired design and at the desired positions on the main body.

As regards electrode contact devices for medical application as implantable electrode contact devices (electrode contact heads and electrode cable attachment devices) in particular, it is of course an important requirement, and consequently an objective, that the size of the respective contact device, that may be produced by the method according to the invention, can be made as small as possible.

Another object is to be able to produce a type of electrode contact device which is well suited for far-reaching "integration" of those parts, portions and areas/zones of the contact device which are to guarantee the mechanical, electrical and physiological functions/properties required of it, so that the device can be manufactured at considerably less cost than has been possible with the technology used to date.

According to the invention, the abovementioned objects are met by using a method for producing an electrode contact device and comprising the steps laid down in the sole patent claim.

An electrode contact device produced by the method according to said patent claim is intended for medical application, and more specifically as a contact device at one end of an electrically insulated conductor or electrode cable for a cardiac pacemaker. The contact device comprises a main body which is made of ceramic material and on the outside of which there is at least one contact portion which is made of electrically conductive material and which is intended to be arranged in electrical communication with a conductor which can be attached to the contact device. The method according to the present invention, which is intended for producing an electrode contact device for medical application as a contact device to be mounted at one end of an electrically insulated conductor or electrode cable for a cardiac pacemaker and having at least two separate contact portions consisting of electrically conductive material, comprises the steps of:
(a) providing a main body of ceramic material;
(b) arranging a layer of electrically conductive material on said main body, and is characterized by the steps of:
(c) arranging a layer of ceramic material on said main body at least partially overlapping said layer of conductive material; and
repeating steps (b) and (c) a selected number of times for producing a selected number of contact portions on said main body, at least two of these contact portions being produced by deposition, e.g. metallization, of electrically conductive material to form said layers.

In practice, it should be possible for any suitable metal to be used as said electrically conductive material. The addition, application or deposition of the material is usually referred to as metallization. The term ceramic material is to be understood in this context to mean inorganic, nonmetallic materials produced by means of a high-temperature reaction. A ceramic which may be suitable in this context is, for example, alumina (Al₂O₃).

An electrode contact device intended for a cardiac pacemaker can be either an electrode contact head at one end (the distal end) of an electrode cable or can be an attachment device arranged at the opposite end (the proximal end) of such an electrode cable. In both cases, the ceramic main body can be provided on the outside with two or more electrically conductive contact portions which are radially and/or axially separated from one another and which each consist of a thin layer of material which has been produced by deposition, or other type of surface application, of electrically conductive material on a particular surface area of the main body, which thin layer of material then forms an electrical contact zone on the outside of the contact device.

The thin conductive layer or layers of material, thus constituting contact surfaces on the outside of the contact device, can be formed easily in the desired manner, and can form contact surface patterns or zones at the optimal locations from the point of view of electrical current transmission.

The contact surface areas can be found both on the jacket surface of the contact device and also on its front end surface.

In some cases there is a requirement for the contact device not only to have extensive contact surface areas, but also to have several punctiform contact portions at some or other part, preferably at the front end surface.

The main body of the contact device will in practice often be designed as a support body which is rotationally symmetrical about its longitudinal axis and which supports the actual conductive surface areas and also any pin-like elements which may optionally be present and which are embedded in the main body. In some cases the contact device may be used as an attachment device arranged at the "pacemaker end" (the proximal end) of an electrode cable,

According to the method of the present invention, the starting point is a core in the form of a main body of ceramic material, on which a layer of electrically conductive material is arranged, after which a layer of ceramic material is arranged on the main body, at least partially overlapping the layer of conductive material. Depending on requirements, it is then possible to apply, in a corresponding manner, alternately a layer of conductive material, a layer of ceramic material, and so on.

The invention will now be described and explained in greater detail hereinbelow, with reference to the attached drawings which show different types of electrode contact devices being at least partly produced by the method according to the present invention.
Figures 1a and 1b are, respectively, an axial longitudinal section through, and an end view from above, of a first embodiment of an electrode contact device which is designed as an electrode contact head;
Figures 2a and 2b are, respectively, a side view, and an end view from above, of an embodiment of a main body for an electrode contact device;
Figures 3a and 3b are, respectively, an axial longitudinal section through, and an end view from above, of an embodiment of a main body for an electrode contact device;
Figures 4a and 4b are, respectively, a side view, and an end view from above, of another embodiment of an electrode contact head; and
Figure 5 shows, in a side view, a contact device designed as an electrode cable attachment device.

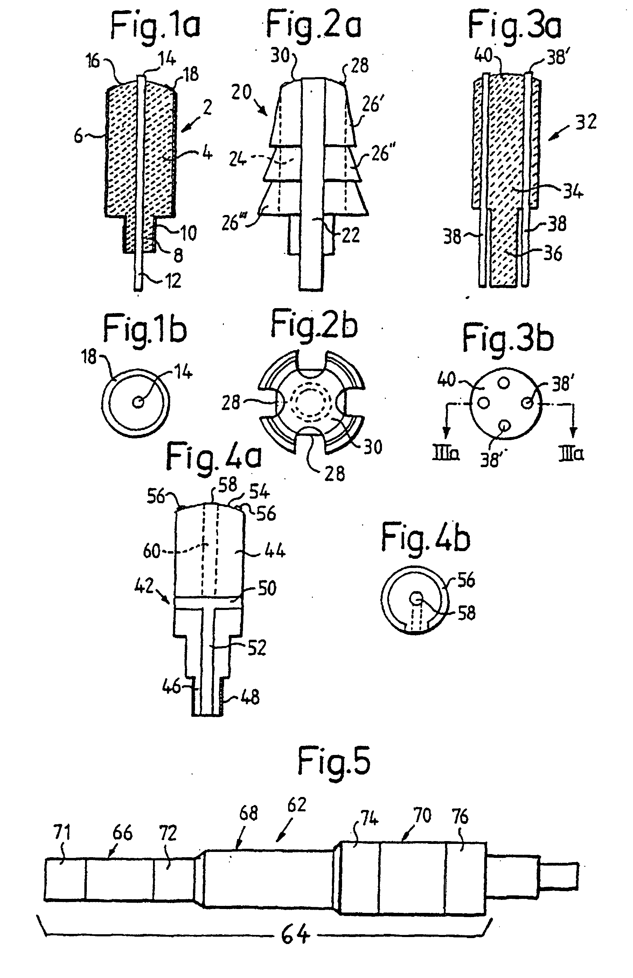

The electrode contact head 2 which is shown in Figure 1 is intended to be arranged at the "heart muscle end" (the distal end) of an electrode cable whose other end (the "pacemaker end", i.e. the proximal end) is attached to a cardiac pacemaker. The electrode contact head 2 comprises an essentially cylindrical main body 4 made of ceramic material. A contact portion 6 consisting of a thin layer of metal is arranged on the outside of the main body 4 and surrounds the circumference of the latter. The main body 4 has, at the bottom, a narrower shaft portion 8 which can be provided with a metallized surface 10. Passing through the ceramic main body 4, in the longitudinal direction of the latter, is a central, axial contact element 12 which is embedded in the main body and whose free end 14 sticks out a short distance from the end surface 16 of the main body 4. At the outer edge of this end surface, the metal layer 6 merges with a circumferential, inwardly directed edge flange 18. The central, axial contact element 12 can be a titanium pin, for example. The metallized contact surfaces 6, 10, 18 of the contact device 2, and also the contact pin 12, are electrically connected in a conventional manner to their respective conductors in the electrode cable (not shown here) which leads to a cardiac pacemaker.

Figure 2 shows a second embodiment of an electrode contact head 20 with four axially extending band-shaped surface areas 22 between which the main body 24 is provided with an axial set of barbs 26', 26'' and 26'''. The contact surfaces 22 which are designed as metallized areas are provided at the top with inwardly directed end tongues 28 on the end surface 30 of the main body.

Figure 3 shows an essentially cylindrical electrode contact head 32 whose ceramic main body 34 merges at the bottom with an axial shaft portion 36 of a smaller diameter than the main body 34. Passing through the ceramic main body 34 in this case are four axially directed contact pins 38 whose free uppermost ends 38' stick out from the upper end surface 40 of the main body 34. The ceramic main body 34 is shown in this case without any thin conductive surface layer on the jacket surface, although one or more electrically conductive surface layers are imagined to be arranged on the main body. In this embodiment too, the four axial contact pins 38 can be titanium pins, for example.

The electrode contact head 42 shown in Figure 4 comprises a rotationally symmetrical main body 44 on whose shaft portion 46 a conductive surface layer 48 is arranged. On the jacket of the main body 44 there is in this case a band-shaped surface area 50 which runs right round and which forms a contact surface area. Connected to this contact surface area there is an axially extending, band-shaped surface area 52. The ceramic main body 44 has in this case, on its upper end surface 54, two contact surface areas 56 and 58 separate from one another. The surface area 56 has the shape of a discontinuous ring, while the surface area 58 has the shape of a radially directed band. The surface area 58 is furthermore in electrical communication with a band-shaped conductor 60 which is integrated or embedded in the ceramic main body 44 and which is indicated by the broken-line contour in Figure 4a.

A common feature of the four electrode contact heads shown in Figures 1 - 4 is that they are multiple-pole contact heads.

Finally, reference is now made to an electrode contact device, shown in Figure 5, which is in the form of an end attachment device 62 intended to be arranged at the "pacemaker end", i.e. the proximal end, of an electrode cable at whose other end (the distal end) there is an electrode contact head. The main body 64 of the end attachment device 62 consists of a ceramic material, and the main body is, as can be seen, a cylindrical body with three axially separated sections or portions 66, 68 and 70 with diameters increasing in steps. The portion 66 is in this case shown with two band-like contact surfaces or contact zones 71 and 72 which extend right round the circumference, while the portion 68 of the main body has no electrically conductive surface layer. In this case, the portion 70 of the main body has two axially separated contact zones 74 and 76. Instead of their two contact zones, it could alternatively be possible for the portions 66 and 70 of the main body each to have a single continuous contact surface layer. If necessary, the portion 68 of the main body could also be provided with some type of conductive metal surface layer.

## Claims

1. A method for producing an electrode contact device for medical application as a contact device to be mounted at one end of an electrically insulated conductor or electrode cable for a cardiac pacemaker and having at least two separate contact portions consisting of electrically conductive material, comprising the steps of:
(a) providing a main body of ceramic material;
(b) arranging a layer of electrically conductive material on said main body;
and **characterized by** the steps of:
(c) arranging a layer of ceramic material on said main body at least partially overlapping said layer of conductive material; and
repeating steps (b) and (c) a selected number of times for producing a selected number of contact portions on said main body, at least two of these contact portions being produced by deposition, e.g. metallization, of electrically conductive material to form said layers.

## Patentansprüche

1. Verfahren zum Herstellen einer Elektrodenkontaktvorrichtung für eine medizinische Anwendung, wie eine an einem Ende eines elektrisch isolierten Leiters oder Elektrodenkabels für einen Herzschrittmacher zu montierende Kontaktvorrichtung, mit wenigstens zwei getrennten, aus elektrisch leitendem Material gebildeten Kontaktabschnitten, umfassend die Schritte:
a) Bereitstellen eines Hauptkörpers aus Keramikmaterial;
b) Anordnen einer Schicht aus elektrisch leitendem Material auf dem Hauptkörper;
und **gekennzeichnet durch** die Schritte:
c) Anordnen einer Schicht aus Keramikrnaterial auf dem Hauptkörper, die wenigstens teilweise die genannte Schicht aus leitendem Material überlappt; und
Wiederholen der Schritte (b) und (c), eine ausgewählte Anzahl von Malen zum Erzeugen einer ausgewählten Anzahl von Kontaktabschnitten auf dem Hauptkörper, wobei wenigstens zwei dieser Kontaktabschnitte **durch** Abscheidung, beispielsweise Metallisierung, eines elektrisch leitenden Materials hergestellt werden, um die genannten Schichten zu bilden.

## Revendications

1. Procédé pour produire un dispositif à contact d'électrode pour une application médicale en tant que dispositif à contact destiné à être monté à une extrémité d'un conducteur ou câble d'électrode isolé électriquement pour un stimulateur cardiaque et ayant au moins deux parties à contact distinctes constituées de matériau conducteur de l'électricité, comportant les étapes qui consistent à :
(a) prendre un corps principal en matériau céramique ;
(b) disposer une couche de matériau conducteur de l'électricité sur le corps principal ;
et **caractérisé par** les étapes qui consistent à :
(c) disposer une couche de matériau en céramique sur le corps principal en chevauchement au moins partiel de la couche de matériau conducteur ; et
répéter les étapes (b) et (c) un nombre de fois sélectionné pour produire un nombre sélectionné de parties de contact du corps principal, au moins deux de ces parties de contact étant produites par dépôt, par exemple par métallisation, de matériau conducteur de l'électricité pour former les couches.
